# EUROPEAN PATENT SPECIFICATION

(11) **EP 3 525 867 B1**
(45) Date of publication and mention of the grant of the patent: **11.02.2026**
(21) Application number: 17791861.2
(22) Date of filing: 17.10.2017
(51) Int. Cl.: A61M 27/00, A61M 25/00, A61F 5/44

(54) **COMPACT URINARY CATHETER WITH PRE-ATTACHED COLLECTION BAG**
KOMPAKTER HARNKATHETER MIT VORMONTIERTEM SAMMELBEUTEL
SONDE URINAIRE COMPACT AVEC POCHE DE COLLECTE PRÉ-FIXÉE

(30) Priority: 17.10.2016 US 201662409115 P
(43) Date of publication of application: 21.08.2019
(60) Divisional application: 20215634.5
(73) Proprietor: Hollister Incorporated, Libertyville, IL 60048 (US)
(72) Inventor: MCMENAMIN, Martin, Libertyville, IL 60048 (US); FLETTER, Paul, C., Libertyville, IL 60048 (US); FOLEY, Adam, J., Libertyville, IL 60048 (US)
(74) Representative: FRKelly
(86) International application number: PCT/US2017/056901
(87) International publication number: WO 2018/075464

(56) References cited:
- EP-A1- 1 023 882
- EP-A1- 2 106 821
- US-A1- 2006 025 753
- US-A1- 2007 225 688

## Description

### CROSS-REFERENCE TO RELATED APPLICATION

This application claims the benefit of and priority to U.S. Provisional Application No. 62/409,115, filed October 17, 2016.

### Field of the Disclosure

The present disclosure is directed to urinary catheters with pre-attached urine collection containers. The present disclosure is further directed to a compact, intermittent urinary catheter that is easy to manipulate, particularly by a user with limited dexterity, without interference from the pre-attached container. The present disclosure is also directed to a compact, intermittent urinary catheter connected to and spaced from a pre-attached urine collection container by a flexible connector.

### Background

Catheters are used to treat many different types of medical conditions and typically include an elongated catheter tube that is inserted into and through a passageway or lumen of the body. Urinary catheters and, in particular, intermittent urinary catheters are commonly used by individuals who suffer from certain abnormalities of the urinary system, such as urinary incontinence. With the advent of intermittent urinary catheters, individuals with problems associated with the urinary system can conveniently self-catheterize to drain the individual's bladder. Individuals who suffer from urinary incontinence will often self-catheterize several times a day.

Self-catheterization involves removing the catheter assembly from its package and inserting and advancing the catheter tube through the user's urethra. Often, urinary catheter assemblies include a urine collection container or bag that must either be attached by the user or is pre-attached to the catheter. In many cases, users of intermittent urinary catheters have limited or diminished dexterity that is often the result of spinal cord injuries. Catheter assemblies with attached urine collection containers may be particularly difficult to manipulate by individuals of limited dexterity as the container is often in close proximity to the catheter and may interfere with the user's ability to comfortably position the catheter or advance the catheter. EP1023882 discloses a urinary catheter assembly comprising a urinary catheter and a flexible tubular catheter package comprising a hose member with a cavity narrowly surrounding the catheter. US2006025753 discloses a urinary catheter assembly that can reduce the need for a user to handle the urinary catheter directly, thereby reducing the likelihood of a urinary tract infection and making the process less messy. US2007225688 discloses a urinary catheter device which provides for a means to lengthen and shorten the tube used to transport urine to a collection receptacle. EP2106821 discloses a catheter assembly comprising a catheter having an insertion end and a discharge end, and a receptacle.

Thus, for these and other reasons, it is desirable that the intermittent catheters and the urine collection containers associated therewith be easy to manipulate and deploy. To that end, it would be desirable to space the pre-attached container away from the catheter tube such that it does not interfere with self-catheterization and helps promote urine flow into the container, and yet is not spaced so far from the catheter that the user must deal with excess tubing and excess material, making the assembly less compact and more cumbersome to manipulate.

### Summary

The invention is defined by claim 1 and further defined by the dependent claims. In one aspect, the present disclosure is directed to a urinary catheter assembly that includes a catheter tube having a proximal end and a distal end. The catheter tube defines a flow path and includes one or more access eyelets at its proximal end. The catheter assembly also includes a urine collection container defining an interior urine collection chamber wherein the container is spaced from the catheter tube by a bendable or otherwise deformable connector. The connector includes a first end and a second end, and defines a flow path between the catheter tube and the collection chamber.

In other aspects, the catheter assembly may include a flow indicator that determines the presence of liquid and/or the rate of liquid flow within the flexible connector or the catheter assembly flow path.

The bendable connector may be, without limitation, a separate polymeric tube with a smooth finish connected to a distal end of the catheter, a flat sheet and/or an extendible and pleated member located between the catheter tube and collection container.

### Brief Description of the Drawings

Figure 1 is a plan view of the catheter assembly of the present disclosure with the pre-attached container in a deployed condition;
Figure 2 is a plan view of the catheter assembly of the present disclosure with the pre-attached container in a folded, packaged condition;
Figure 3 is a perspective view of one embodiment of the catheter assembly disclosed herein with an attachment fixture for attaching the connector to the catheter;
Figure 3A is an enlarged, partial view of the catheter assernbly of Fig.3 at the point of attachment of the catheter to the connector;
Figure 4 is an enlarged plan view of the attachment fixture of Fig. 3 described above;
Figure 5 is a plan view of the attachment fixture of Figs. 3-3A connected to the proximal end of the connector;
Figure 6 is a plan view of the attachment fixture connected to the connector and ready for attachment to the gripping member of the catheter;
Figure 7 is a partial perspective view of the catheter assembly disclosed herein with an alternative embodiment of a connector;
Figure 8 is an end view of the connector of Figure 7 in a collapsed, no flow condition;
Figure 9 is an end view of the connector of Figure 7 in an expanded, flow condition;
Figure 10 is a plan view of the catheter assembly of Fig. 1 with a flow indicator;
Figure 11 is a partial plan view of the another embodiment of the catheter assembly of the present disclosure prior to mounting of a gripping member;
Figure 12 is a partial plan view of the catheter assembly of Fig. 1 1 with the gripping member mounted thereon;
Figure 13 is a perspective view of the catheter assembly of the present disclosure with a further alternative embodiment of a flexible connector;
Figure 14 is a perspective view of the catheter assembly of Fig. 6 in a non-deployed configuration;
Figure 15 is a perspective view of the catheter assembly of Fig. 6 with the flexible connector in a deployed configuration;
Figure 16 is a cross-sectional side view of a pleated connector in a bent condition; and
Figure 17 is a cross-sectional side view of the pleated connector of Fig. 16 n an extended condition.

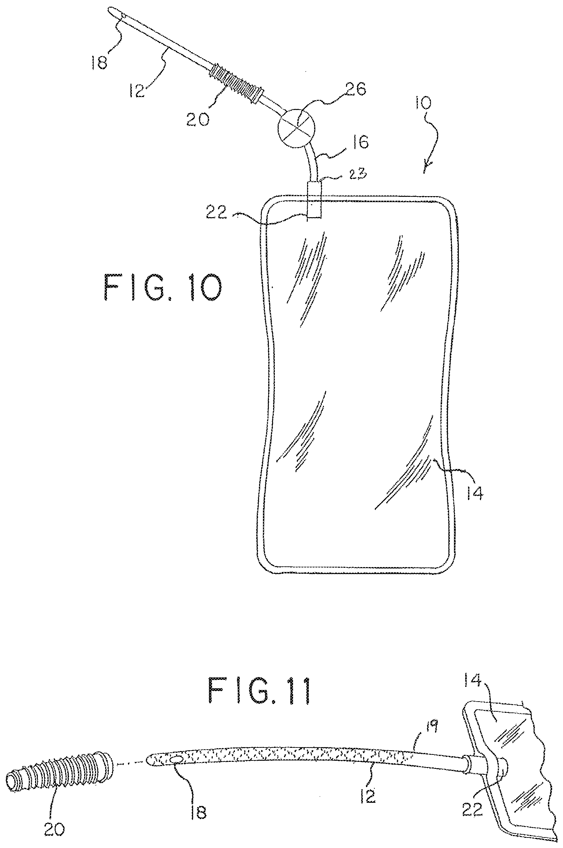

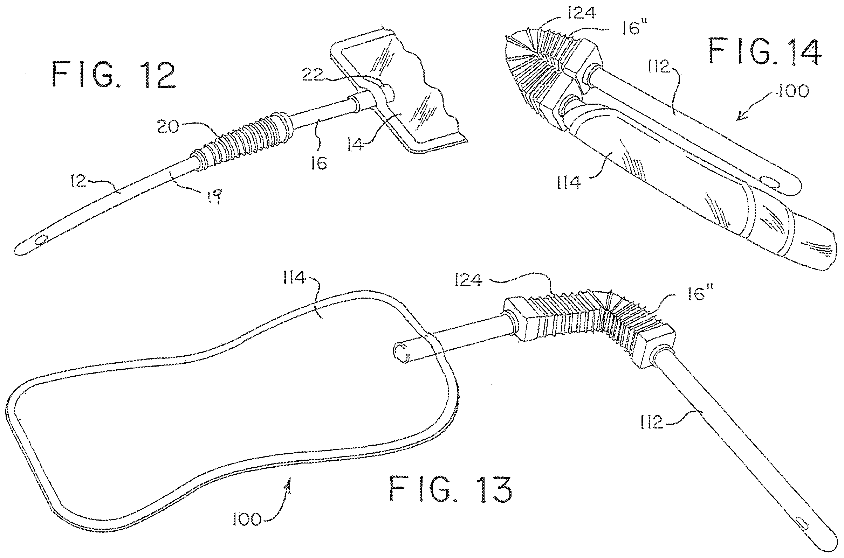

Figures 11 and 12 do not depict catheter assemblies of the invention but rather catheter assemblies representing background useful for the understanding of the invention.

### Detailed Description of the Embodiments

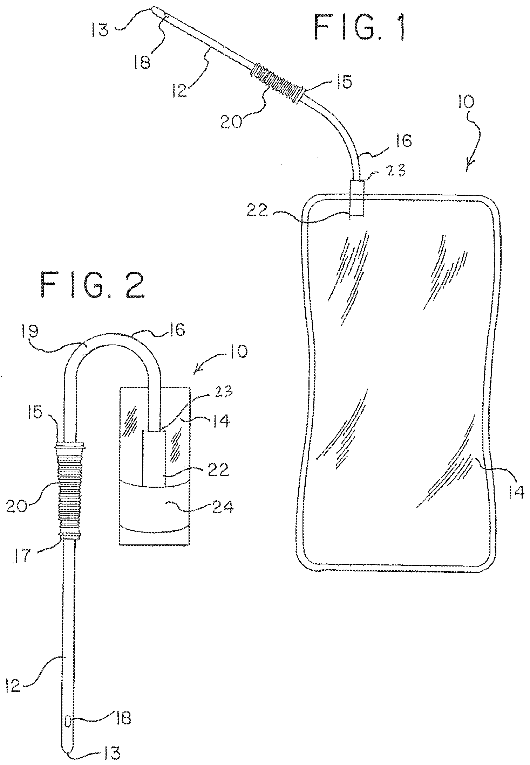

With reference to the Figures, Fig. 1 shows one example of catheter assembly 10 in accordance with the present disclosure. Catheter assembly 10 includes a catheter tube 12 for insertion into the urethra of the patient and a container 14 for collecting urine discharged from the bladder of the patient. Catheter tube 12 includes a proximal end 13 which terminates in a tip adapted for insertion into the urethra of the patient. Catheter tube 12 includes one or more access eyelets 18 located near tip 13 through which urine enters the flow path 19 of tube 12. Catheter tube 12 is attached at its distal end 17 to a gripping member 20. Catheter assembly 10 further includes a connector 16 between gripping member 20 and container 14. As shown in the Figures, connector 16 spaces container 14 away from distal end 17 of catheter tube 12 and gripping member 20. This allows the user to more easily manipulate, position and/or insert catheter tube 12 during self-catheterization without container 14 interference, during self- catheterization by the user. As shown in Fig. 2, prior to use, container 14 may be folded and packaged into a compact catheter assembly package. A strip of tape 24 or band of paper or other securing member may hold folded container 14 and catheter 12 in its compact configuration during storage and prior to use.

Spacing container 14 at a distance from the catheter also allows container 14 to be positioned vertically lower than catheter 12, which aids in the flow of urine into container 12 during catheterization (due to the declivity). As container 14 fills and becomes heavier and more difficult to grasp, the flexibility of connector 16 allows container 14 to be placed a selected distance from catheter, making the catheterization process less cumbersome by not having to keep container 14 close to catheter 12.

Accordingly, connector 16 should be of sufficient length to allow for adequate spacing of catheter tube 12 from container 14 and allow independent movement of catheter tube 12 relative to container 14. In that regard, connector 16 should not be so long that it includes excess tubing as one would typically encounter with catheter assemblies employing a "leg bag". In accordance with the present disclosure, connector 16 should be of sufficient length to allow for easy manipulation of catheter tube 12 without interference from collection container 14. Container 14 should be freely movable with movement of connector 16. An example of a suitable connector 16 length is, for example approximately 5 to approximately 10 cm.

As shown in the Figures and discussed below, connector 16 may preferably be an elongated, separate member that is attached to the catheter tube and the container, respectively. In another embodiment, discussed in connection with Figs. 11-12 below, connector 16 may be provided as a portion of an elongated catheter tube 12 which receives and carries a gripping member 20 at a selected location along tube 12.

Flexible connector 16 defines flow path 19 (Fig. 2) between catheter tube 12 and container 14. Urine entering catheter tube 12 through eyelets 18 flows through the catheter tube, through the flexible connector 16, and into container 14. Collection container 14 may be any type of container that is suitable for use in the urinary catheter field. Details of the container are beyond the scope of the present application and will not be discussed further.

As further shown, catheter assembly 10 includes a gripping member 20. Gripping member 20 (which may also serve as a funnel) provides a flow conduit for the urine during self- catheterization. As seen in Figs. 1-3, gripping member 20 includes a proximal end, which is directly attached to distal end 17 of catheter tube 12, and an open distal end 15. Connector 16 is attached to gripping member 20 at open distal end 15 of gripping member 20. Opposite end 23 of flexible connector 16 is joined to container 14 either directly or through a connector port 22, as discussed below.

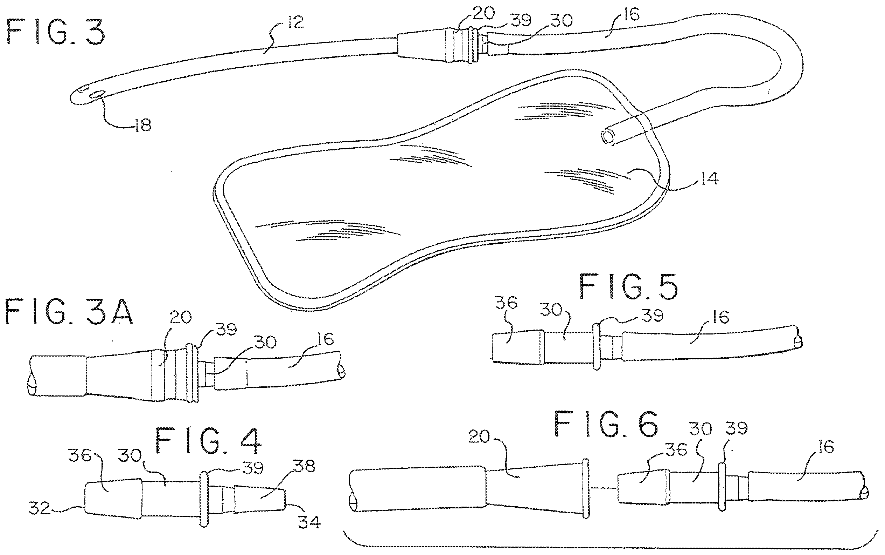

In one embodiment, connector 16 may be joined to gripping member 20 by providing a port (not shown) that may be attached to gripper 20 , by spin welding, as generally described in U.S. Application Publication No. 2015/0105756, which is incorporated by reference. Other bonding techniques, such as solvent bonding, may also be used to attach proximal end of connector 16 to open end 15 of gripping member 20. For example, in one embodiment, connector 16 may be attached to gripping member 20 (and ultimately catheter 12) by an attachment fixture 30 as shown, for example, Figs. 3-6. As shown in Fig. 3, attachment fixture 30 may be a molded article made of a polymeric material, having a proximal end 32 adapted for connection with open distal end 15 of gripper 20 and a distal end 34, adapted for connection with open proximal end of connector 16. Fixture 30 defines a flow path between proximal and distal ends 32, 34. As further shown in Fig. 4, the external diameter of fixture 30 may be variable, being greater at proximal end 32 than at distal end 34.

Continuing with the description of fixture 30, proximal and/or distal end 32, 34 may each respectively, include barbs 36 and 38, Barbs 36 and/or 38 may typically be provided as integral portions of fixture proximal and distal ends 32, 34 of fixture 30 and have greater outer diarneter(s) than adjacent portions of the respective proximal/distal ends 32, 34. As shown in Fig. 3, barbs 36 and/or 38 may be tapered and allow for press-fit attachment to gripping member 20 and connector 16. For example, proximal barb 36 may be inserted into open end 15 of gripper 20 and advanced to achieve a secure attachment of high tensile strength. In one embodiment, barb 36 may be advanced until ring 39 on fixture 30 contacts gripping member 20, as shown in Fig. 3A. Similarly, if fixture includes a barb at each end thereof, distal barb 38 may be inserted and press-fit into the open proximal end of connector 16. Fixture 30 may alternatively include a single, two-sided barb that is secured to both the gripping member 20 and connector 16. Fixture 30 may be made of any suitable polymeric material that can be molded. Non-limiting example of such materials include acrylonitrile butadiene styrene (ABS), polyvinyl chloride, polycarbonate. Fixtures of the type described herein are available from Qosina of Ronkonkoma, NY.

As discussed above, connector 16 may be joined to container 14 either directly (as generally depicted in Fig. 3 by sealing container walls directly to the outer surface of connector 16) or through connector port 22 by any one of the methods described above used to join connector 16 to gripper 20 (or catheter 12) i.e., spin welding, solvent bonding or barb attachment.

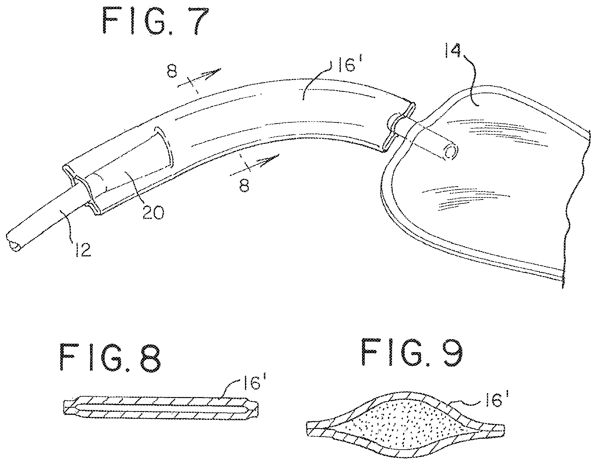

Connector 16, shown in Figs. 1-3, may be made of any polymeric material that is suitable for medical use. Connector 16 may, for example, be made of polyvinyl chloride or other suitable flexible or bendable material. Connector 16 is preferably more flexible than catheter tube 12 and more easily bendable (as shown in Figs. 2 and 9). Typically, connector 16 has a hardness of less than Shore GDA.

The catheter assembly described herein where catheter tube 12 (with gripper 20) connector 16 and collection container are all pre-attached, prior to use, provides a closed urine catheterization and collection apparatus. In an alternative embodiment, collection container 14 and connector 16 may be fully integrated with the connector being attached (e.g., press fit using a barb) to gripper 20 by the user just prior to catheterization.

Where visual detection of flow is desired, connector 16 may be sufficiently transparent and/or sufficiently flat. For example, in the embodiment of Figs. 1-6, connector 16 may be a bendable, substantially transparent tube. In another embodiment, connector 16 may be a flat (and preferably transparent) sleeve, which changes shape during fluid flow and reverts to a flat shape when flow has ceased, as shown in Figs. 7-9. Figure 7 shows connector 16' as substantially flat sleeve. As fluid moves through connector 16', it changes shape (e.g., bulges) as shown in Fig. 9, indicating to the user, the presence of fluid In a further variant, connector 16' may allow for detection of flow by using an internal valve, such as a flutter valve. In particular, connector may include a rubber sleeve (not shown) within a bendable connector tube 16 or connector sleeve 16'. Changes in the shape of the rubber sleeve indicate the presence of flow through the transparent wall of connector 16, 16".

Fig. 10 shows an alternative embodiment of catheter assembly 10. In the embodiment of Fig. 10, catheter assembly 10 may include a dedicated flow indicator 26, which may be employed and/or associated with catheter assembly 10. Flow indicator 26 may be a sensor that is used to determine the presence of liquid and/or determine the rate of fluid flow within connector 16. Flow indicator 26 may be particularly useful in a closed, intermittent catheter system such as the one described herein. Users of such systems often require extra time self-catheterizing to ensure that their bladder is completely empty. While initial flow upon introduction of the catheter into the urethra may be indicated visually as urine fills the collection container, near the end of the catheterization process, continued flow may be difficult to perceive. When most of the urine has been drained into the collection container and only a small volume remains in the bladder, the flow velocity decreases, and the weight of the bag does not noticeably change as it does in the beginning. Accordingly, the user may be unaware that flow has stopped. Without any reliable confirmation that flow has ceased, users may maintain the intermittent catheter in place longer than is necessary. This adds unneeded and undesired time to the process of self-catheterization.

Other forms of visual detection of fluid flow may also serve to indicate the presence of flow. For example, flow indicator 26 may be provided with a gauge, a needle, a spinning paddle wheel, plastic spheres that move or spin or the like, in response to flow.

In addition to the above, an electro/mechanical flow indicator 26 (generically depicted in Fig. 10) may be provided as part of catheter assembly 10. Flow indicator 26 may be either analog or digital. Flow detector 26 may operate under the principles of optical detection of fluid flow, differential pressure, changes, mass flow, thermal changes, velocity changes, or positive displacement. Flow indicator 26 may also include logic and memory circuitry or other smart technology.

In addition, flow indicator 26 may not only monitor the presence and flow of fluid, but may also analyze the composition of the urine for purposes of monitoring bladder health and/or compliance with treatments, therapy, or rehabilitation protocols. Flow indicator 26 may allow for wireless digital connection, which can provide alerts on mobile devices and the like.

Figs. 11 and 12 show a different embodiment of the catheter assembly of the present disclosure not falling within the scope of the appended claims, not forming part of the invention. In the embodiment of Figs. 11-12, spacing of catheter tube 12 from urine collection container 14 is accomplished by providing an extended catheter tube 12. Thus, unlike the embodiment described above, catheter tube and flexible connector 16 make up one integrated piece or section of tubing that defines flow path 19. Gripping member 20, may be slidingly placed onto integrated catheter tube 12/flexible connector, as shown in Fig. 12. Gripping member 20 may be fixed to catheter tube 12/flexible connector 16 at any desired location along the integrated tube. This allows the user to manipulate catheter tube 12 without the interference of container 14, which remains spaced from the catheter tube.

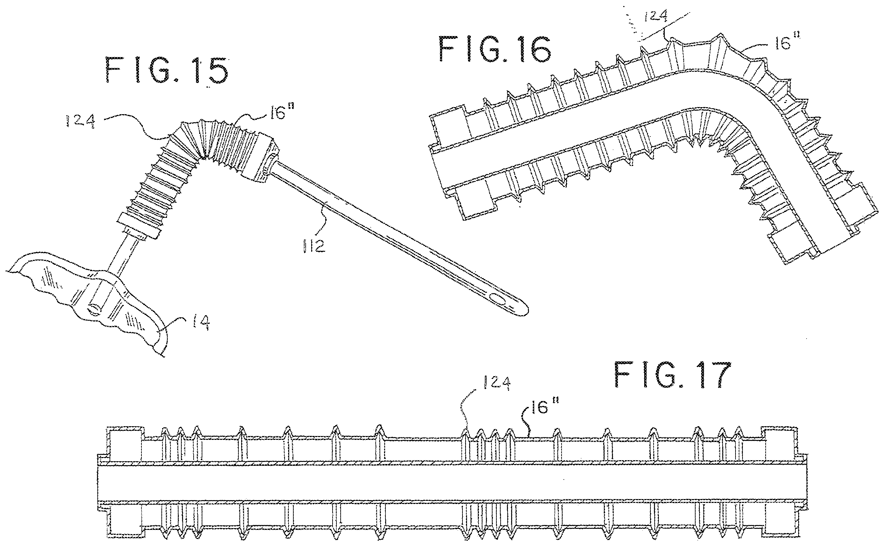

Turning now to Figs. 13-17, a further alternative of catheter assembly 10 is shown. In Figs 13-17, catheter assembly 100 includes a catheter tube 112, a urine collection container 114, and a connector 16". As shown in Figs. 13-17, rather than a smooth, flexible tube or sleeve of fixed length, connector 16" may be a flexible, pleated member that is both extendable along the central axis of connector 16" and can also be bent or deformed relative to its central axis to facilitate insertion and manipulation of catheter tube 112 during self-catheterization. Connector 16" may be bent approximately 180° such that the catheter and undeployed container 114 can be placed side-by-side during storage and prior to use, as shown in Fig. 14. As further seen in Fig. 13-17, connector 16" may be provided in the form of a corrugated or pleated length of bendable and deformable material. Pleats 124 on the outer surface of flexible connector also allow a convenient gripping surface for the user. Connector 16" includes and defines an internal flow path to facilitate flow from catheter tube 112 to flexible container 114. Connector 16" may be made of any resilient material that allows for movement in three dimensions, such as extension/compression (Fig. 17), bending (Fig. 16) and twisting. Examples of suitable materials for use as flexible connector include, but are not limited to polyvinyl chloride.

It should be understood that various changes and modifications to the embodiments described herein will be apparent to those skilled in the art. Such changes and modifications can be made without departing from the scope of the invention as defined by the appended claims.

## Claims

1. A urinary catheter assembly (10) comprising:
a) a catheter tube (12) having a proximal end (13) and a distal end (17), said catheter tube (12) defining a flow path (19) and including one or more access eyelets (18) at said proximal end (13) and a gripping member (20) at said distal end (17), said gripping member (20) having an open distal end (15);
b) a urine collection container (14) spaced from said catheter tube (12) by a deformable connector (16), said urine collection container (14) comprising a urine collection chamber; and
c) said connector (16) comprising a first end and a second end, wherein said flexible connector (16) defines a flow path (19) between said catheter tube (12) and said chamber and wherein said first end of said connector (16) is bonded to said open distal end (15) of said gripping member (20) and said second end is configured for attachment to said container (14) either directly or through a connection port.

2. The urinary catheter assembly (10) of Claim 1 wherein said connector (16) comprises a flexible polymeric tube.

3. The urinary catheter assembly (10) of any one of Claims 1 and 2 wherein the connector (16) comprises a compressible and extendable connector.

4. The urinary catheter assembly (10) of any one of Claims 1 through 3 wherein said connector (16) is bendable 180° without kinking.

5. The urinary catheter assembly (10) of any one of Claims 1 through 4 wherein said flexible connector (16) comprises a pleated outer wall defining said flow path (19).

6. The urinary catheter assembly (10) of any one of Claims 1 through 5 further comprising a flow indicator (26).

7. The urinary catheter assembly (10) of Claim 6 wherein said flow indicator (26) is configured to detect the rate of flow through said flexible connector (16).

8. The urinary catheter assembly (10) of any one of Claims 1 through 7 wherein said connector (16) comprises an attachment fixture (30) defining a flow path wherein said fixture (30) is configured for attaching said connector (16) to said open distal end (15) of said gripping member (20).

9. The urinary catheter assembly (10) of any one of Claims 1 through 8 wherein said gripping member (20) comprises a funnel.

10. The urinary catheter assembly (10) of any one of Claims 1 through 9 wherein said first end of said connector (16) is solvent bonded to said gripping member (20).

11. The urinary catheter assembly (10) of any one of Claims 10 through 11 wherein said connector (16) comprises one or more barbs (36, 38) configured for attachment to said gripping member (20) and/or configured for attachment to said container (14).

12. The urinary catheter assembly (10) of any one of Claims 1 through 12 further comprising a connection port on said urine collection container (14) configured for attachment to a distal end of said flexible connector (16).

13. The urinary catheter assembly (10) of any one of Claims 1 through 13 wherein said catheter tube (12) has a stiffness, and said connector (16) has a stiffness that is different from said catheter tube (12).

14. The urinary catheter assembly (10) of any one of Claims 1 through 14 wherein said connector (16) comprises a shaft with a central axis wherein said connector (16) is configured for movement in three dimensions relative to said central axis.

## Patentansprüche

1. Harnkathetanordnung (10), umfassend:
a) einen Katheterschlauch (12) mit einem proximalen Ende (13) und einem distalen Ende (17), wobei der Katheterschlauch (12) einen Durchlauf (19) definiert und an seinem proximalen Ende (13) eine oder mehrere Zugangsösen (18) sowie an seinem distalen Ende (17) ein Greifelement (20) aufweist, wobei das Greifelement (20) ein offenes distales Ende (15) aufweist;
b) einen Harnauffangbehälter (14), der durch ein verformbares Verbindungsstück (16) von dem Katheterschlauch (12) beabstandet ist und der Harnauffangbehälter (14) eine Harnauffangkammer aufweist; und
c) wobei der Verbinder (16) ein erstes und ein zweites Ende aufweist, wobei der flexible Verbinder (16) einen Durchlauf (19) zwischen dem Katheterschlauch (12) und der Kammer definiert und wobei das erste Ende des Verbinders (16) mit dem offenen distalen Ende (15) des Greifelements (20) verbunden ist und das zweite Ende zur Befestigung an dem Behälter (14) entweder direkt oder über eine Verbindungsöffnung konfiguriert ist.

2. Harnkatheteranordnung (10) nach Anspruch 1, wobei der Verbinder (16) einen flexiblen Polymerschlauch umfasst.

3. Harnkatheteranordnung (10) nach einem der Ansprüche 1 und 2, wobei der Verbinder (16) einen komprimierbaren und ausziehbaren Verbinder umfasst.

4. Harnkatheteranordnung (10) nach einem der Ansprüche 1 bis 3, wobei der Verbinder (16) um 180° biegbar ist, ohne abzuknicken.

5. Harnkatheteranordnung (10) nach einem der Ansprüche 1 bis 4, wobei der flexible Verbinder (16) eine gefaltete Außenwand aufweist, die den Durchlauf (19) definiert.

6. Harnkatheteranordnung (10) nach einem der Ansprüche 1 bis 5, ferner umfassend eine Durchlaufanzeige (26).

7. Harnkatheteranordnung (10) nach Anspruch 6, wobei die Durchlaufanzeige (26) so konfiguriert ist, dass sie die Durchlaufrate durch den flexiblen Verbinder (16) erfasst.

8. Harnkatheteranordnung (10) nach einem der Ansprüche 1 bis 7, wobei der Verbinder (16) eine Befestigungsvorrichtung (30) umfasst, die einen Durchlauf definiert, wobei die Vorrichtung (30) so konfiguriert ist, dass der Verbinder (16) an das offene distale Ende (15) des Greifelements (20) angeschlossen werden kann.

9. Harnkatheteranordnung (10) nach einem der Ansprüche 1 bis 8, wobei das Greifelement (20) einen Trichter umfasst.

10. Harnkatheteranordnung (10) nach einem der Ansprüche 1 bis 9, wobei das erste Ende des Verbinders (16) mit dem Greifelement(20) durch Lösungsmittel verbunden ist.

11. Harnkatheteranordnung (10) nach einem der Ansprüche 10 bis 11, wobei der Verbinder (16) einen oder mehrere Widerhaken (36, 38) aufweist, die zur Befestigung an dem Greifelement (20) und/oder zur Befestigung an dem Behälter (14) konfiguriert sind.

12. Harnkatheteranordnung (10) nach einem der Ansprüche 1 bis 12 ferner umfassend einen Anschluss am Harnauffangbehälter (14), der zur Befestigung an einem distalen Ende des flexiblen Verbindungsstücks (16) ausgelegt ist.

13. Harnkatheteranordnung (10) nach einem der Ansprüche 1 bis 13, wobei der Katheterschlauch (12) eine Steifigkeit aufweist und der Verbinder (16) eine von dem Katheterschlauch (12) unterschiedliche Steifigkeit aufweist.

14. Harnkatheteranordnung (10) nach einem der Ansprüche 1 bis 14, wobei der Verbinder (16) einen Schaft mit einer Mittelachse umfasst, wobei der Verbinder (16) so konfiguriert ist, dass er sich in drei Dimensionen relativ zu dieser Mittelachse bewegen kann.

## Revendications

1. Ensemble de sonde urinaire (10) comprenant :
a) un tube de sonde (12) présentant une extrémité proximale (13) et une extrémité distale (17), ledit tube de sonde (12) définissant un chemin d'écoulement (19) et comportant un ou plusieurs œillets d'accès (18) au niveau de ladite extrémité proximale (13) et un élément de préhension (20) au niveau de ladite extrémité distale (17), ledit élément de préhension (20) présentant une extrémité distale ouverte (15) ;
b) un récipient de collecte d'urine (14) espacé dudit tube de sonde (12) par un raccord déformable (16), ledit récipient de collecte d'urine (14) comprenant une chambre de collecte d'urine ; et
c) ledit raccord (16) comprenant une première extrémité et une seconde extrémité, dans lequel ledit raccord flexible (16) définit un chemin d'écoulement (19) entre ledit tube de sonde (12) et ladite chambre et dans lequel ladite première extrémité dudit raccord (16) est liée à ladite extrémité distale ouverte (15) dudit élément de préhension (20) et ladite seconde extrémité est configurée pour la fixation audit récipient (14) soit directement, soit par l'intermédiaire d'un orifice de raccordement.

2. Ensemble de sonde urinaire (10) selon la revendication 1 dans lequel ledit raccord (16) comprend un tube polymère flexible.

3. Ensemble de sonde urinaire (10) selon l'une quelconque des revendications 1 et 2 dans lequel le raccord (16) comprend un raccord compressible et extensible.

4. Ensemble de sonde urinaire (10) selon l'une quelconque des revendications 1 à 3 dans lequel ledit raccord (16) est pliable à 180° sans formation de coude.

5. Ensemble de sonde urinaire (10) selon l'une quelconque des revendications 1 à 4, dans lequel ledit raccord flexible (16) comprend une paroi extérieure plissée définissant ledit chemin d'écoulement (19).

6. Ensemble de sonde urinaire (10) selon l'une quelconque des revendications 1 à 5 comprenant également un indicateur d'écoulement (26).

7. Ensemble de sonde urinaire (10) selon la revendication 6 dans lequel ledit indicateur d'écoulement (26) est configuré pour détecter le débit d'écoulement à travers ledit raccord flexible (16).

8. Ensemble de sonde urinaire (10) selon l'une quelconque des revendications 1 à 7, dans lequel ledit raccord (16) comprend un dispositif de fixation (30) définissant un chemin d'écoulement dans lequel ledit dispositif (30) est configuré pour fixer ledit raccord (16) à ladite extrémité distale ouverte (15) dudit élément de préhension (20).

9. Ensemble de sonde urinaire (10) selon l'une quelconque des revendications 1 à 8 dans lequel ledit élément de préhension (20) comprend un entonnoir.

10. Ensemble de sonde urinaire (10) selon l'une quelconque des revendications 1 à 9 dans lequel ladite première extrémité dudit raccord (16) est collée par solvant audit élément de préhension (20).

11. Ensemble de sonde urinaire (10) selon l'une quelconque des revendications 10 à 11 dans lequel ledit raccord (16) comprend une ou plusieurs ergots (36, 38) configurés pour être fixés audit élément de préhension (20) et/ou configurés pour être fixés audit récipient (14).

12. Ensemble de sonde urinaire (10) selon l'une quelconque des revendications 1 à 12 comprenant également un orifice de raccordement sur ledit récipient de collecte d'urine (14) configuré pour être fixé à une extrémité distale dudit raccord flexible (16).

13. Ensemble de sonde urinaire (10) selon l'une quelconque des revendications 1 à 13 dans lequel ledit tube de sonde (12) présente une rigidité et ledit raccord (16) présente une rigidité différente de celle dudit tube de sonde (12).

14. Ensemble de sonde urinaire (10) selon l'une quelconque des revendications 1 à 14 dans lequel ledit raccord (16) comprend une tige munie d'un axe central dans lequel ledit raccord (16) est configuré pour se déplacer dans trois dimensions par rapport audit axe central.
